# EUROPÄISCHE PATENTSCHRIFT

(11) **EP 3 344 111 B1**
(45) Veröffentlichungstag und Bekanntmachung des Hinweises auf die Patenterteilung: **03.07.2019**
(21) Anmeldenummer: 16812670.4
(22) Anmeldetag: 29.08.2016
(51) Int. Cl.: A61B 1/00, A61B 1/05

(54) **SCHAFTINSTRUMENT UND INSBESONDERE MEDIZINISCH-ENDOSKOPISCHES SCHAFTINSTRUMENT**
SHAFT INSTRUMENT AND IN PARTICULAR A MEDICAL ENDOSCOPIC SHAFT INSTRUMENT
INSTRUMENT À TIGE, EN PARTICULIER INSTRUMENT MÉDICAL D'ENDOSCOPIE À TIGE

(30) Priorität: 03.09.2015 DE 102015216864
(43) Veröffentlichungstag der Anmeldung: 11.07.2018
(73) Patentinhaber: Richard Wolf GmbH, 75438 Knittlingen (DE)
(72) Erfinder: FREY, Sebastian, 68753 Waghäusel (DE); HÄHNLE, Friedrich, 75015 Bretten (DE); KÖRNER, Eberhard, 75438 Knittlingen (DE)
(74) Vertreter: Patentanwälte Vollmann Hemmer Lindfeld Partnerschaft mbB
(86) Internationale Anmeldenummer: PCT/DE2016/200402
(87) Internationale Veröffentlichungsnummer: WO 2017/036479

(56) Entgegenhaltungen:
- US-A1- 2002 049 367
- US-A1- 2008 108 869
- US-A1- 2009 292 164
- US-B1- 8 403 826

## Beschreibung

Die Erfindung betrifft ein Schaftinstrument und insbesondere ein medizinisch-endoskopisches Schaftinstrument.

Im Bereich der minimal-invasiven Chirurgie werden standardmäßig starre, semiflexible oder flexible Schaftinstrumente eingesetzt, die durch einen natürlichen oder künstlich geschaffenen Zufuhrkanal in das Körperinnere eines zu behandelnden Patienten eingeführt werden und dort zur Untersuchung von Hohlräumen bzw. Hohlorganen verwendet werden. Instrumente dieser Art finden auch auf technischem Gebiet als sogenannte Technoskope Verwendung, wo sie in schwer zugänglichen Hohlräumen technischer Objekte eingesetzt werden.

Zur optischen Begutachtung eines Hohlraums bzw. eines Hohlorgans weisen diese Schaftinstrumente eine Beobachtungseinrichtung und eine Beleuchtungseinrichtung zur Ausleuchtung des Beobachtungsbereichs auf. Als Beobachtungseinrichtungen kommen entweder sich von dem distalen Ende des Schafts durch den Schaft erstreckende und proximalseitig des Schafts mit Okularen in Verbindung stehende Linsensysteme oder an dem distalen Ende des Schafts angeordnete elektronische Bildsensoren einer Videokamera zum Einsatz, wobei die Bildsensoren mit einer proximalseitig des Schafts angeordneten Bilddatenverarbeitungseinrichtung und einer dieser Einrichtung nachgeordneten Betrachtungseinrichtung verbunden sind. Die Beleuchtungseinrichtungen umfassen entweder zumindest einen durch den Schaft geführten und proximalseitig des Schafts mit einer Lichtquelle verbundenen Lichtleiter oder weisen mindestens ein an dem distalen Ende des Schafts angeordnetes lichtemittierendes Halbleiterelement auf, das an einer proximalseitig des Schafts angeordneten Spannungsquelle angeschlossen ist.

Den Ausgangspunkt der Erfindung bilden solche Schaftinstrumente, die neben einer optischen Beobachtung auch die Durchführung operativer Eingriffe in dem Beobachtungsgebiet ermöglichen. Bei diesen Schaftinstrumenten werden Hilfsinstrumente, wie beispielsweise Zangen oder Schneidinstrumente durch den als Hohlschaft ausgebildeten Schaft zu dem Operationsgebiet geführt. Hierbei bestimmt der am distalen Ende des Schafts in dem Schaft seitlich neben den dort angeordneten Teilen der Beobachtungs- und der Beleuchtungseinrichtung zur Verfügung stehende Freiraum maßgeblich die Art der verwendbaren Hilfsinstrumente, da deren Abmessungen mit dem vorhandenen Freiraum in dem Schaft korrespondieren müssen. Dies bedeutet speziell, dass Hilfsinstrumente, deren radiale Abmessungen in ihrem durch den Schaft geführten Bereich eine gewisse Größe übersteigen, nicht eingesetzt werden können. Zwar besteht die Möglichkeit, dieser Einschränkung durch Bereitstellung eines Hohlschafts mit einem größeren Durchmesser entgegen zu wirken, allerdings führt diese Maßnahme dazu, dass bei einem medizinisch-endoskopisch eingesetzten Schaftinstrument gegebenenfalls eine deutlich gesteigerte Traumatisierung von Körpergewebe in Kauf genommen werden muss und das Einsatzspektrum des Schaftinstruments generell verringert wird, da es in Körperhöhlen bzw. Hohlorganen, die lediglich über einen sehr engen Zugang erreichbar sind, aufgrund der Größe seines Schaftdurchmessers gar nicht verwendet werden kann.

Aus EP 2123225 A1 zählt es zwar zum Stand der Technik, ein im Bereich des distalen Schaftendes angeordnetes Kameragehäuse aus dem Innenlumen des Schaftes herauszufahren, doch ausschließlich aus optischen Gründen, um eine für den Eingriff günstigere Beobachtungsposition zu erhalten. Der Arbeitskanal ist hiervon nicht tangiert, die Ausfahrmechanik ist aufwändig, raumbeanspruchend und anfällig.

Aus US 5,166,787 A zählt es zum Stand der Technik, am distalen Schaftende ein Kameragehäuse ausschwenkbar anzuordnen. Diese Anordnung schafft zwar einen Freiraum im Arbeitskanal, ist jedoch hinsichtlich der Betätigungsmechanik und Kabelführung kritisch.

Vor diesem Hintergrund liegt der Erfindung die Aufgabe zugrunde, ein Schaftinstrument und insbesondere ein medizinisch-endoskopisches Schaftinstrument zu schaffen, welches die vorab beschriebenen Nachteile nicht aufweist.

Diese Aufgabe wird durch ein Schaftinstrument mit den in Anspruch 1 angegeben Merkmalen gelöst, wobei sich vorteilhafte Weiterbildungen dieses Instruments aus den Unteransprüchen, der nachfolgenden Beschreibung sowie der Zeichnung ergeben. Hierbei können die in den Unteransprüchen angegebenen Merkmale vorteilhaft in der angegebenen Kombination aber auch, soweit technisch sinnvoll, für sich oder in anderer Kombination zur Ausgestaltung der Erfindung beitragen.

Das erfindungsgemäße Schaftinstrument ist bevorzugt ein medizinisch-endoskopisches Schaftinstrument, es kann sich bei ihm aber auch um ein im technischen Bereich eingesetztes Technoskop handeln. Das Schaftinstrument ist mit einem Hohlschaft ausgestattet, durch welchen bei Bedarf ein Hilfsinstrument und/oder ein Fluid bis nach distalseitig des Hohlschafts geführt werden kann. Der Hohlschaft kann sowohl starr als auch zumindest abschnittsweise in Richtung quer zu seiner Längsausdehnung flexibel biegbar ausgebildet sein.

Ferner weist das Schaftinstrument mindestens einen elektronischen Bildsensor und Leuchtmittel auf, wobei der zumindest eine Bildsensor dazu dient, einen distalseitig des Hohlschafts befindlichen Bereich optisch zu erfassen und die Leuchtmittel dafür vorgesehen sind, diesen von dem Bildsensor erfassten Bereich auszuleuchten. Als Bildsensor können alle bekannten elektronischen Bildsensoren verwendet werden, wobei als Halbleiter-Chips ausgebildete Bildsensoren, wie beispielsweise CMOS-Sensoren oder CCD-Sensoren wegen ihrer geringen Baugröße bevorzugt werden. Als Leuchtmittel sind vorzugsweise lichtemittierende Halbleiterelemente, wie Leucht- oder Laserdioden vorgesehen, die sich ebenfalls durch ihre geringe Baugröße auszeichnen.

Bei dem erfindungsgemäßen Schaftinstrument sind der zumindest eine Bildsensor und/oder die Leuchtmittel in einem distalseitig in dem Hohlschaft integrierten Gehäuse angeordnet, wobei unter dem Begriff "Gehäuse" erfindungsgemäß alle den Bildsensor bzw. die Leuchtmittel außenseitig umgebenden Ummantelungen, also auch einfache Beschichtungen zu verstehen sind. Ausgehend von dem Gehäuse ist ein darin angeordneter Bildsensor mit einer vorzugsweise proximalseitig des Hohlschafts angeordneten Bilddatenverarbeitungseinrichtung signalverbunden, welche wiederum mit einer Betrachtungseinrichtung in Signalverbindung steht. Die zur Ausleuchtung vorgesehenen Leuchtmittel sind über durch den Hohlschaft geführte Stromleiter mit einer ebenfalls proximalseitig des Hohlschafts angeordneten Spannungsquelle leitungsverbunden. Bevorzugt sind in einem gemeinsamen Gehäuse sowohl ein Bildsensor als auch zumindest ein lichtemittierendes Halbleiterelement als Leuchtmittel angeordnet.

Das erfindungsgemäße Schaftinstrument zeichnet sich dadurch aus, dass das Gehäuse mit dem darin angeordneten Bildsensor und/oder den darin angeordneten Leuchtmitteln von einer Stellung, in welcher der Bildsensor und/oder die Leuchtmittel innerhalb des Innenlumens des Hohlschafts, das heißt innerhalb dessen Lichtraumprofils angeordnet sind, geradlinig in eine Stellung bewegbar ist, in welcher der Bildsensor und/oder die Leuchtmittel außerhalb des Innenlumens des Hohlschafts angeordnet sind.

Unter Innenlumen im Sinne der vorliegenden Erfindung ist das vom Schaft umschlossene Volumen zu verstehen, also das durch seine Innenkontur bestimmte.

Das Schaftinstrument kann somit einen ersten Betriebszustand aufweisen, in dem das Gehäuse vollständig innerhalb des Außenprofils des Hohlschafts angeordnet ist, und von dort durch eine lineare Verschiebung des Gehäuses in einen zweiten Betriebszustand überführt werden, in dem das Gehäuse zum größten Teil seitlich außerhalb des Hohlschafts angeordnet ist. In seinem ersten Betriebszustand kann der Hohlschaft des Schaftinstrument über vergleichsweise enge Zufuhrkanäle zu seinem Bestimmungsort in einem Hohlorgan oder Hohlraum geführt werden, wobei sich der Bereich distalseitg des Hohlschafts mittels des Bildsensors und/oder der Leuchtmittel, die dann innerhalb des Innenlumens des Hohlschafts angeordnet sind, beobachten und/oder ausleuchten lässt. Anschließend kann das Gehäuse in einer geradlinigen Bewegung vorzugsweise in radialer Richtung des Hohlschafts aber ggf. auch schräg zur Längsausdehnung des Hohlschafts weitestgehend aus dem Innenlumen des Hohlschafts herausbewegt werden, wobei auch dann eine Beobachtung und/oder Ausleuchtung des Bereichs distalseitig des Hohlschafts gewährleistet ist und besonders vorteilhaft der freie Raum zur Durchführung eines Hilfsinstruments bzw. zur Leitung eines Fluids durch den Hohlschaft erheblich vergrößert wird. Hierdurch steigert sich die Palette der in Verbindung mit dem Schaftinstrument einsetzbaren Hilfsinstrumente bzw. es eröffnet sich auch die Möglichkeit, die radialen Abmessungen des Hohlschafts des Schaftinstruments ohne Einschränkung der Palette der bislang einsetzbaren Hilfsinstrumente zu verringern. Die geradlinige Bewegbarkeit des Gehäuses ist insofern von Vorteil, als diese konstruktiv und herstellungstechnisch einfach zu realisieren ist und dazu führt, dass der Bildsensor und/oder die Leuchtmittel sowohl im ersten als auch im zweiten Betriebszustand des Schaftinstruments immer gleich, das heißt ohne eine Winkeländerung bezüglich einer Mittelachse des Hohlschafts ausgerichtet sind.

Um das Gehäuse von einer in dem Hohlschaft integrierten Stellung in eine Stellung zu bewegen, in welcher das Gehäuse weitestgehend radial außenseitig des Hohlschafts angeordnet ist, ist an der Umfangswandung des Hohlschafts eine Öffnung ausgebildet, durch welche das Gehäuse verschiebbar ist. Die Lage und Abmessungen dieser Öffnung korrespondieren hierbei zweckmäßigerweise mit der Lage und den Abmessungen des Gehäuses sowie gegebenenfalls mit dem Bewegungsweg des Gehäuses, wobei zwischen dem Gehäuse und der Öffnung nur ein geringes Spiel besteht, so dass das Gehäuse und der Hohlschaft eine möglichst dichte Einheit bilden.

Dabei stützt sich das Gehäuse gemäß der Erfindung an seiner von der an der Umfangswandung des Hohlschafts ausgebildeten Öffnung entgegengesetzt abgewandten Außenseite an mindestens einem in dem Hohlschaft geführten länglichen Träger ab, der sich im Wesentlichen über die gesamte Länge des Hohlschafts erstreckt und im Bereich des proximalen Endes des Hohlschafts oder proximalseitig des Hohlschafts festgelegt ist. Um in dem Innenlumen des Hohlschafts einen möglichst großen Freiraum zur Durchführung eines Hilfsinstruments durch den Hohlschaft bereitstellen zu können, kann der Träger im Bereich des proximalen Endes des Hohlschafts zweckmäßigerweise in möglichst großem radialen Abstand von der Mittelachse des Hohlschafts festgelegt sein.

Dabei ist der Träger elastisch federnd ausgebildet, sodass der Träger bei Einwirkung einer entsprechenden äußeren Kraft in Richtung quer zu seiner Längsausdehnung verbiegbar ist und nach Wegfall dieser Kraft wieder seine ursprüngliche Form annimmt. Dieses elastisch federnde Verhalten des Trägers ist besonders zweckmäßig, da der Träger fest mit dem Gehäuse verbunden ist und auf diese Weise einer Bewegung des Gehäuses von innerhalb des Innenlumens des Hohlschafts nach außerhalb des Innenlumens und umgekehrt ohne eine Beschädigung folgen kann.

In diesem Zusammenhang erweist es sich auch als vorteilhaft, wenn, wie es gemäß einer bevorzugten Weiterbildung der Erfindung vorgesehen ist, eine außenliegende Außenseite des Gehäuses in der Stellung des Gehäuses innerhalb des Innenlumens des Hohlschafts mit der Außenseite der Umfangswandung des Hohlschafts bündig fluchtet. Demnach sind die Lage des innerhalb des Innenlumens des Hohlschafts angeordneten Gehäuses und die Kontur der außenliegenden Außenseite des Gehäuses vorzugsweise so gewählt, dass das innerhalb des Innenlumens des Hohlschafts angeordnete Gehäuse mit dem Hohlschaft eine gemeinsame geschlossene Oberfläche bildet.

Gemäß einer vorteilhaften Weiterbildung der Erfindung ist vorgesehen, dass an dieser innen liegenden Außenseite des Gehäuses, welche von der an der Umfangswandung des Hohlschafts ausgebildeten Öffnung weg weist, eine in Längsrichtung des Hohlschafts verlaufende Ausnehmung ausgebildet ist, in die der Träger eingreift. Das Ziel dieser Maßnahme besteht darin, dass sich der Träger bei aus dem Innenlumen des Hohlschafts herausbewegten Gehäuse in dem Hohlschaft möglichst wenig bzw. gar nicht auf die Größe des dann geschaffenen vergrößerten Freiraums zur Durchführung eines Hilfsinstruments auswirkt. Hierzu ist die Ausnehmung zweckmäßigerweise so dimensioniert, dass der Träger in der Querschnittsebene des Hohlschafts nicht aus dem Gehäuse herausragt.

Der Träger wird von einem Flachrohr gebildet. Insbesondere im Hinblick auf eine elastische Verbiegbarkeit des Trägers quer zu seiner Längsausdehnung ist das Flachrohr sinnvollerweise derart ausgerichtet, dass sich das Gehäuse an einer Flachseite des Flachrohrs abstützt. Die Flachseite des Flachrohrs ist hierbei normal zu der Bewegungsrichtung des Gehäuses ausgerichtet. Zwar ist die Querschnittsform des als Träger verwendeten Flachrohrs generell beliebig, solange es in einer bestimmten Querschnittsrichtung größere Abmessungen als in einer hierzu normal ausgerichteten Querschnittsrichtung aufweist, allerdings ist es von Vorteil, wenn das Flachrohr einen nierenförmigen Querschnitt aufweist. Hierbei stützt sich das Gehäuse für den Bildsensor und/oder die Leuchtmittel bevorzugt an einer konvex gewölbten Flachseite des Flachrohrs ab, deren Wölbung mit der Innenquerschnittskontur des Hohlschafts korrespondiert, so dass das Flachrohr zumindest in seinem distalen Endabschnitt bei aus dem Innenlumen des Hohlschafts herausbewegtem Gehäuse an dem Innenumfang des Hohlschafts bündig zur Anlage kommt und sich auf diese Weise in einem geringen Maße auf den zur Durchführung des Hilfsinstruments benötigten Freiraum innerhalb des Hohlschafts auswirkt. Die konkav gewölbte Flachseite des nierenförmigen Flachrohrs ist hierbei von der an der Umfangswandung des Hohlschafts ausgebildeten Öffnung entgegengesetzt abgewandt, was sich innerhalb des Hohlschafts ebenfalls positiv hinsichtlich eines möglichst großen Freiraums für die durch den Hohlschaft geführten Hilfsinstrumente auswirkt, da diese Hilfsinstrumente im Regelfall einen Schaft mit einem kreisförmigen Querschnitt aufweisen.

Weiter sind die an dem Bildsensor und/oder der Leuchtdiode angeschlossenen Anschlussleitungen durch das Flachrohr nach proximalseitig des Hohlschafts geführt. Zweckmäßigerweise ist hierbei das Flachrohr an seinem distalen Ende verschlossen ausgebildet, so dass die elektrischen Anschlussleitungen vollständig von einem ggf. durch das freie Innenlumen des Hohlschafts geleiteten Fluids abgeschirmt werden. Bei mehreren durch das Flachrohr geführten Anschlussleitungen, sind diese sinnvollerweise in größtmöglichem Abstand voneinander, also in Richtung der größten Querschnittsbreite des Flachrohrs voneinander beabstandet geführt. Zudem können in dem Flachrohr an dessen distalen Ende vorteilhaft zusätzliche Leuchtmittel angeordnet sein, die eine besonders helle Ausleuchtung des von dem Bildsensor erfassten Bereichs bewirken.

Zur Bewegungssteuerung des Gehäuses für den Bildsensor und/oder die Leuchtmittel, das heißt zur Steuerung der geradlinigen Bewegung des Gehäuses von einer Stellung, in welcher der Bildsensor und/oder die Leuchtmittel innerhalb des Innenlumens des Hohlschafts angeordnet sind, in eine Stellung, in welcher der Bildsensor und/oder die Leuchtmittel außerhalb des Innenlumens des Hohlschafts angeordnet sind, ist das Gehäuse vorteilhaft über eine Kulissensteuerung mit einem in dem Hohlschaft in Längsrichtung des Hohlschafts bewegbaren Schub-Zug-Element bewegungsgekoppelt. Hierbei wird eine Bewegung des proximalseitig des Hohlschafts mit einer Betätigungseinrichtung wirkungsverbundenen Schub-Zug-Elements in Längsrichtung des Hohlschafts über die Kulissensteuerung in eine Bewegung des Gehäuses quer zur Längsrichtung des Hohlschafts umgesetzt.

Zur Bildung der Kulissensteuerung weist das Schub-Zug-Element bevorzugt zumindest einen quer zu seiner Bewegungsrichtung ausgerichteten Vorsprung auf, der in eine an einer Außenseite des Gehäuses ausgebildete und in radialer Richtung schräg verlaufende Führungsnut eingreift. Wird das Schub-Zug-Element in Längsrichtung des Hohlschafts bewegt, bewirkt die an seiner Außenseite schräg verlaufende Führungsnut und der darin eingreifende Vorsprung des Schub-Zug-Elements eine zwangsgeführte Bewegung des Gehäuses in radialer Richtung quer zur Längsausdehnung des Hohlschafts bzw. quer zur Bewegungsrichtung des Schub-Zug-Elements.

Insbesondere im Hinblick auf die Schaffung eines möglichst großen Freiraums zur Durchführung eines Hilfsinstruments durch den Hohlschaft erweist es sich aber als vorteilhaft, wenn das Schub-Zug-Element von einem Rohr gebildet wird, welches sinnvollerweise möglichst dünnwandig ausgebildet ist und einen mit dem Innendurchmesser des Hohlschafts korrespondierenden Außendurchmesser aufweist. In diesem Fall wird das Hilfsinstrument durch das in dem Hohlschaft angeordnete Rohr durch den Hohlschaft geführt. An dem das Schub-Zug-Element bildenden Rohr ist zweckmäßigerweise ein Längsschlitz ausgebildet, in welchen das Gehäuse für den Bildsensor und/oder die Leuchtmittel eingreift. Zudem ist an den beiden Längsseiten des Längsschlitzes jeweils ein Vorsprung ausgebildet. Diese beiden an den Längsseiten des Längsschlitzes ausgebildeten Vorsprünge greifen in zwei an voneinander entgegengesetzt abgewandten Außenseiten des Gehäuses ausgebildete Führungsnuten ein.

Nachfolgend ist die Erfindung anhand von in der Zeichnung dargestellten Ausführungsbeispielen näher erläutert. In der Zeichnung zeigt schematisch vereinfacht und in unterschiedlichen Maßstäben:
- Fig. 1: in perspektivischer Darstellung einen distalen Endabschnitt eines medizinisch-endoskopischen Schaftinstruments in einem ersten Betriebszustand,
- Fig. 2: die Darstellung nach Fig. 1 mit dem Schaftinstrument in einem zweiten Betriebszustand,
- Fig. 3: in vergrößerter Darstellung einen distalen Endbereich eines Schaft des Schaftinstruments nach Fig. 1 in dem ersten Betriebszustand,
- Fig. 4: in vergrößerter Darstellung einen distalen Endbereich des Schafts des Schaftinstruments nach Fig. 1 in dem zweiten Betriebszustand,
- Fig. 5: in einer Frontansicht den distalen Endabschnitt des Schaftinstruments nach Fig. 1 in dem ersten Betriebszustand,
- Fig. 6: in einer Frontansicht den distalen Endabschnitt des Schaftinstruments nach Fig. 1 in dem zweiten Betriebszustand und
- Fig. 7: in vergrößerter Darstellung einen distalen Endbereich eines Schafts eines Schaftinstruments gemäß einer zweiten Ausgestaltung in dem zweiten Betriebszustand.

Die in der Zeichnung dargestellten Schaftinstrumente weisen jeweils einen von einem starren Rohr gebildeten Hohlschaft 2 auf, an dem sich proximalseitig des Hohlschafts 2 ein Gehäuseteil 4 anschließt. Im Bereich des distalen Endes 6 des Hohlschafts 2 ist in dem Hohlschaft 2 ein Gehäuse 8 integriert. An einer distalen Stirnseite 10 des Gehäuses 8 ist eine Ausnehmung 12 ausgebildet, in welcher ein Bildsensor 14 angeordnet ist. Seitlich der Ausnehmung 12 sind an der Stirnseite 10 des Gehäuses 8 zwei weitere Ausnehmungen 16 ausgebildet, in denen jeweils eine Leuchtdiode 18 als Leuchtmittel angeordnet ist. In dem Gehäuse 8 sind der Bildsensor 14 und die Leuchtdiode 18 austauschbar angeordnet, so dass der Bildsensor 14 und die Leuchtdioden 18 bei Bedarf aus dem Gehäuse 18 ausgebaut werden können und durch einen anderen Bildsensor 14 bzw. durch andere Leuchtdioden 18 ersetzt werden können.

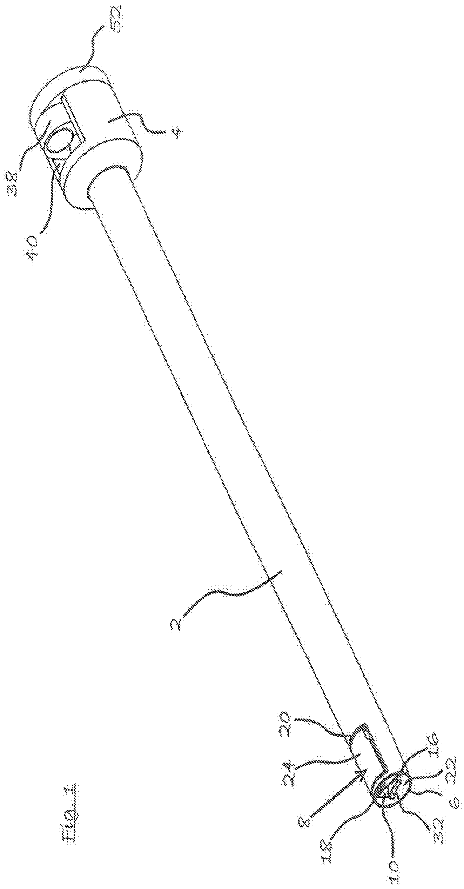

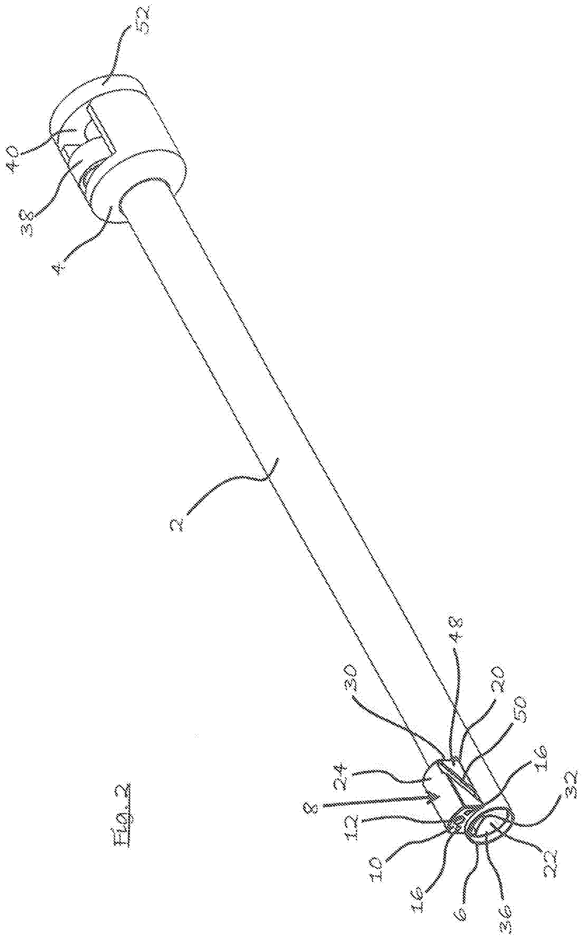

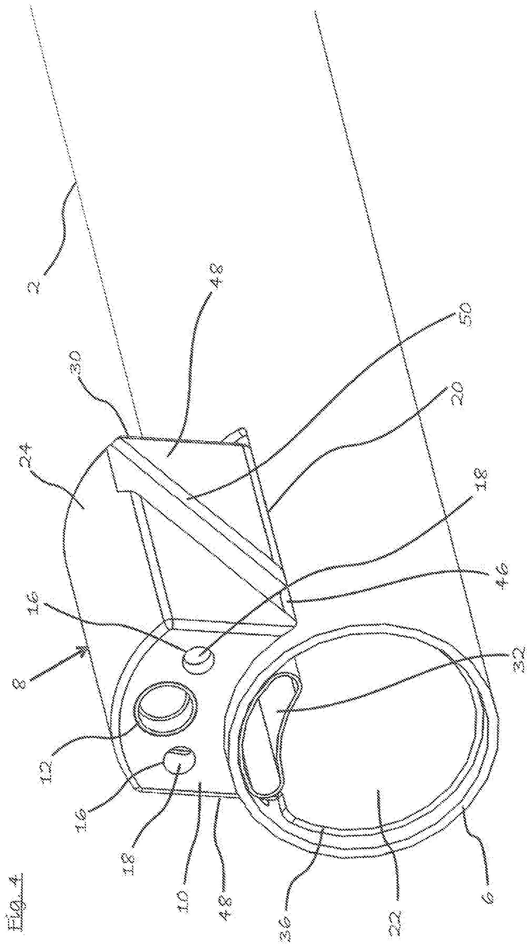

Durch eine an der Umfangswandung des Hohlschafts 2 ausgebildete Öffnung 20 ist das Gehäuse von einer Stellung, in welcher der Bildsensor 24 und die Leuchtdioden 8 innerhalb des Innenlumens 22 des Hohlschafts 2 angeordnet sind (Fig. 1, 3 und 5) in eine Stellung bewegbar, in welcher der Bildsensor 14 und die Leuchtdioden 18 außerhalb des Innenlumens 22 des Hohlschafts 2 angeordnet sind (Fig. 2, 4, 6 und 7). In der Stellung, in welcher der Bildsensor 14 und die Leuchtdioden 18 innerhalb des Innenlumens 22 des Hohlschafts 2 angeordnet sind, fluchtet eine außenliegende Außenseite 24 des Gehäuses 8, welche eine zu der Außenrundung des Hohlschafts 2 komplementäre Wölbung aufweist, mit der Außenseite des Hohlschafts 2.

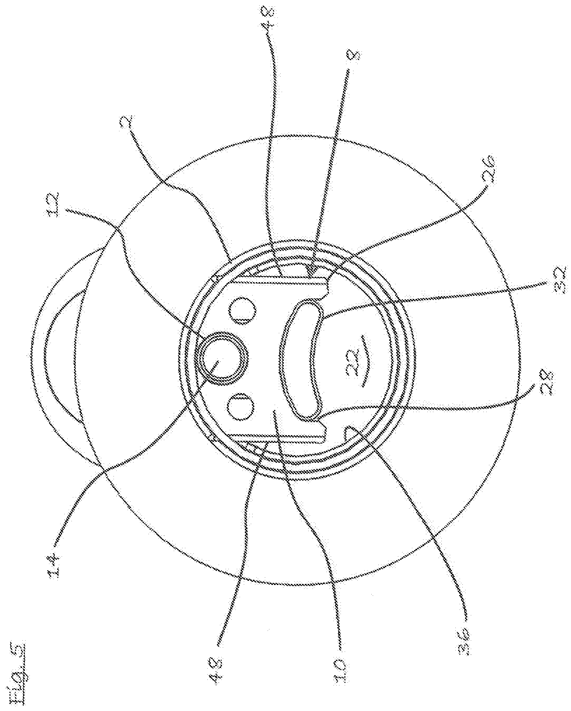

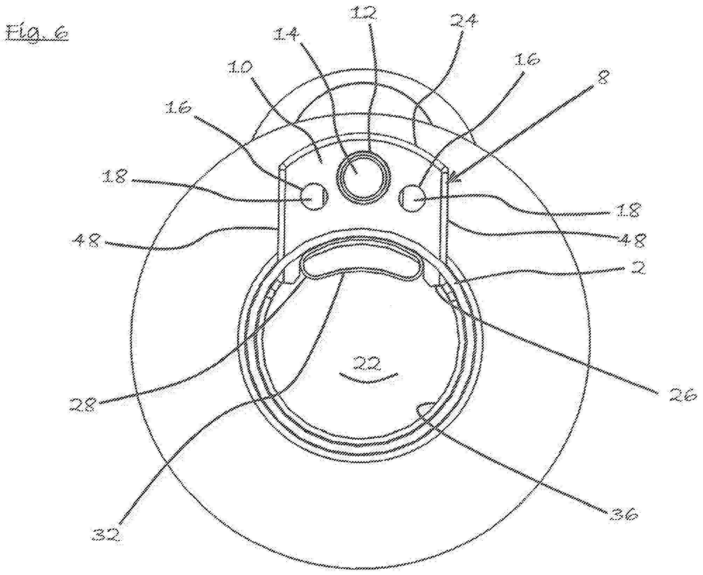

Wie insbesondere aus den Fig. 5 und 6 deutlich wird, ist an einer von der Öffnung 20 bzw. von der Außenseite 24 des Gehäuses 8 abgewandten innenliegenden Außenseite 26 des Gehäuses 8 eine Ausnehmung 28 ausgebildet. Diese Ausnehmung 28 erstreckt sich in Längsrichtung des Hohlschafts 2 von der distalen Stirnseite 10 zu einer proximalen Stirnseite 30 des Gehäuses 8 und dient zur Aufnahme eines Flachrohrs 32 mit einem nierenförmigen Querschnitt, welches sich von dem distalen Ende des Hohlschafts 2 bis proximalseitig des Hohlschafts 2 erstreckt und proximalseitig des Hohlschafts 2 an dem Gehäuseteil 40 unbeweglich festgelegt ist. Das Flachrohr 32 bildet einen Träger, auf dem sich das Gehäuse 8 sowohl in seiner Stellung innerhalb des Innenlumens 22 des Hohlschafts 2 als auch in seiner aus den Innenlumen 22 herausbewegten Stellung abstützt. Um der Bewegung des Gehäuses 8 folgen zu können, weist das Flachrohr 32 eine gewisse Elastizität in Richtung quer zu seiner Längsausdehnung auf so dass es elastisch quer zu seiner Längsausdehnung verbogen werden kann.

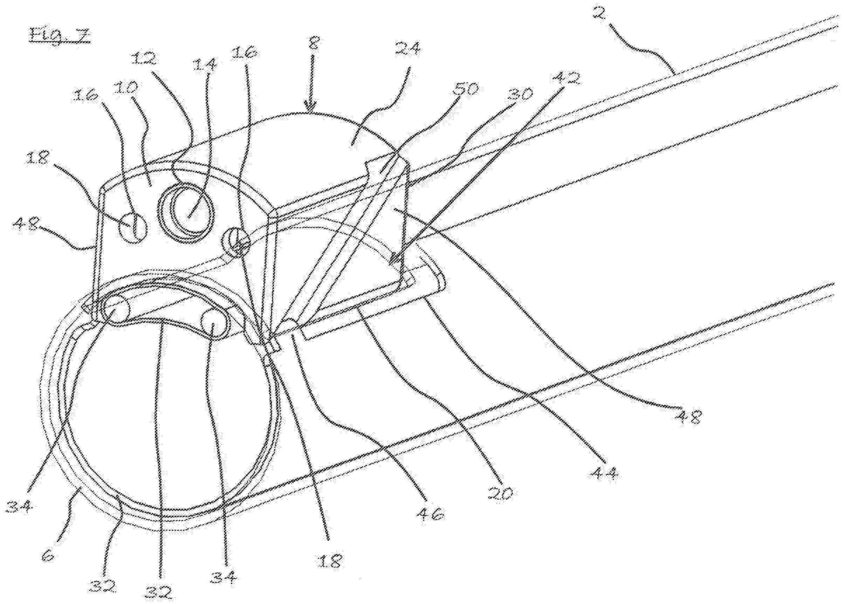

Neben seiner Funktion als Träger für das Gehäuse 8 dient das Flachrohr 32 auch als Führungskanal für an dem Bildsensor 14 und den Leuchtdioden 18 angeschlossene, in der Zeichnung aus Übersichtlichkeitsgründen nicht dargestellte Anschlussleitungen, die durch das Flachrohr 32 zu einem proximalseitig des Gehäuseteils 4 angeordnetem Gehäuseteil 52 geführt sind, wobei die an dem Bildsensor 14 angeschlossene Anschlussleitung proximalseitig des Gehäuseteils 52 an eine nicht dargestellte Bilddatenverarbeitungseinrichtung und die an den Leuchtdioden 18 angeschlossenen Anschlussleitungen proximalseitig des Gehäuseteils 52 an eine ebenfalls nicht dargestellte Spannungsquelle angeschlossen sind. Bei dem in Fig. 7 dargestellten Ausführungsbeispiel dient das Flachrohr 32 zudem zur Anordnung von zwei zusätzlichen Leuchtdioden 34, die eine besonders helle Ausleuchtung eines von dem Bildsensor 14 erfassten Bereichs distalseitig des Hohlschafts 2 bewirken. Die Verwendung der Leuchtdioden 34 stellt im Übrigen den einzigen Unterschied zwischen dem in Fig. 7 dargestellten Schaftinstrument und dem in den Fig. 1 - 6 dargestelltem Schaftinstrument dar. Zudem sei darauf hingewiesen, dass in dem Flachrohr 32, dort wo die Leuchtdioden 34 angeordnet sind, anstelle der Leuchtdioden 34 Rohre zur Leitung einer Spülflüssigkeit einsetzbar sind, wodurch eine "continous flow"-Spülung möglich wäre.

In dem Hohlschaft 2 ist ein Rohr 36 angeordnet, welches sich bis in das Gehäuseteil 52 erstreckt. Das Rohr 36 ist in dem Hohlschaft 2 in Längsrichtung des Hohlschafts 2 verschiebbar und bildet auf diese Weise ein Schub-Zug-Element. Zur Bewegungssteuerung des Rohrs 36 ist an dessen proximalen, in das Gehäuseteil 4 eingreifenden Endabschnitt ein mit dem Rohr 36 fest verbundener Schieber 38 angeordnet, der an dem Außenumfang des Rohres 36 radial auskragt und eine an dem Gehäuseteil 4 umfänglich ausgebildete Durchbrechung 40 durchgreift. Die Durchbrechung 40 ist so dimensioniert, dass sie eine begrenzte Verschiebung des Schiebers 38 in der Durchbrechung 40 erlaubt (Fig. 1 und 2).

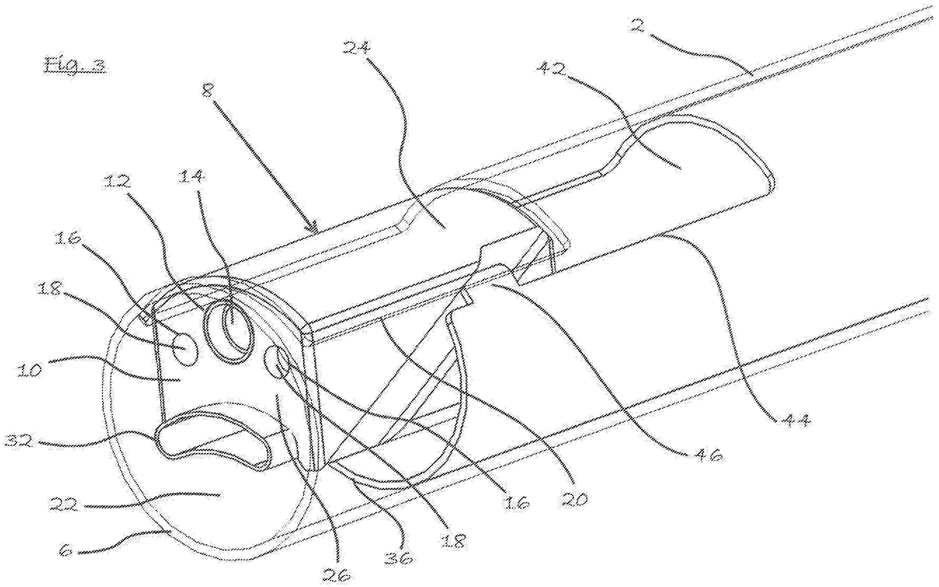

Das Rohr 36 dient als Betätigungselement, mit dem das Gehäuse 8 über eine Kulissensteuerung von einer Stellung, in welcher der Bildsensor 14 und die Leuchtdioden 18 innerhalb des Innenlumens 22 des Hohlschafts 2 angeordnet sind, in eine Stellung bewegbar ist, in welcher der Bildsensor 14 und die Leuchtdioden 18 außerhalb des Innenlumens 22 des Hohlschafts 2 angeordnet sind. Zur Bildung der Kulissensteuerung ist an dem Rohr 36 ausgehend von dessen distalen Ende ein Längsschlitz 42 ausgebildet, der sich in begrenztem Maße in proximaler Richtung des Rohrs 36 erstreckt (Fig. 3 und 7). Die Breite des Längsschlitzes 42 ist so bemessen, dass das Gehäuse 8 den Längsschlitz 42 mit geringem Spiel durchbrechen kann. An den beiden einander gegenüberliegend angeordneten und in axialer Richtung des Längsschlitzes 42 verlaufenden Längsseiten 44 des Längsschlitzes 42 ist jeweils ein Vorsprung 46 ausgebildet, welcher quer zur Längsausdehnung des Längsschlitzes 42 hervorragt. Die beiden Vorsprünge 46 bilden Kulissensteine, die in Kulissen an den Gehäuse 8 eingreifen. Zur Bildung dieser Kulissen ist an zwei normal zu dem Längsschlitz 42 des Rohrs 36 ausgerichteten Außenseiten 48 des Gehäuses 8 jeweils eine Führungsnut 50 ausgebildet, welche sich ausgehend von dem proximalen Ende des Außenseite 24 des Gehäuses 8 schräg zu dem distalen Ende der Außenseite 26 des Gehäuses 8 erstreckt. In jeder der beiden Führungsnuten 50 ist jeweils einer der Vorsprünge 46 in Eingriff. Wird das Rohr 36 durch entsprechende Betätigung des Schiebers 38 in distaler Richtung verschoben, bewirkt der Eingriff der an dem Rohr 36 ausgebildeten Vorsprünge 46 in die schräg ausgerichteten Führungsnuten 50 des Gehäuses 8, dass das Gehäuse 8 von einer Stellung, in welcher der Bildsensor 14 und die Leuchtdioden 18 innerhalb des Innenlumens 22 des Hohlschafts 2 angeordnet sind, in eine Stellung bewegt wird, in welcher der Bildsensor 14 und die Leuchtdioden 18 außerhalb des Innenlumens 22 des Hohlschafts angeordnet sind. Bei einer Verschiebung des Rohrs 36 in proximaler Richtung wird das Gehäuse 8 dann wieder in den Hohlschaft 2 eingefahren.

### Bezugszeichenliste

- 2: Hohlschaft
- 4: Gehäuseteil
- 6: Ende
- 8: Gehäuse
- 10: Stirnseite
- 12: Ausnehmung
- 14: Bildsensor
- 16: Ausnehmung
- 18: Leuchtdiode
- 20: Öffnung
- 22: Innenlumen
- 24: Außenseite
- 26: Außenseite
- 28: Ausnehmung
- 30: Stirnseite
- 32: Flachrohr
- 34: Leuchtdiode
- 36: Rohr
- 38: Schieber
- 40: Durchbrechung
- 42: Längsschlitz
- 44: Längsseite
- 46: Vorsprung
- 48: Außenseite
- 50: Führungsnut
- 52: Gehäuseteil

## Patentansprüche

1. Schaftinstrument, insbesondere medizinisch-endoskopisches Schaftinstrument, mit einem Hohlschaft (2) und mit zumindest einem distalseitig in dem Hohlschaft (2) integrierten Gehäuse (8) mit mindestens einem darin angeordneten elektronischen Bildsensor (14) zur optischen Erfassung eines distalseitig des Hohlschafts (2) befindlichen Bereichs und/oder darin angeordneten Leuchtmitteln zur Ausleuchtung dieses Bereichs, wobei das Gehäuse (8) von einer Stellung, in welcher der Bildsensor (14) und/oder die Leuchtmittel innerhalb des Innenlumens (22) des Hohlschafts (2) angeordnet sind, geradlinig in eine Stellung bewegbar ist, in welcher der Bildsensor (14) und/oder die Leuchtmittel außerhalb des Innenlumens (22) des Hohlschafts (2) angeordnet sind, wobei das Gehäuse (8) durch eine an der Umfangswandung des Hohlschafts (2) ausgebildete Öffnung (20) verschiebbar ist und an seiner von der an der Umfangswandung des Hohlschafts (2) ausgebildeten Öffnung (20) entgegengesetzt abgewandten innenliegenden Außenseite (26) an mindestens einem in dem Hohlschaft (2) geführten länglichen und quer zu seiner Längsausdehnungen elastisch federnd ausgebilden Träger abstützt, welcher sich im Wesentlichen über die gesamte Länge des Hohlschafts (2) erstreckt und im Bereich des proximalen Endes des Hohlschafts (2) oder proximalseitig des Hohlschafts (2) festgelegt ist **dadurch gekennzeichnet, dass** der Tröger von einem Flachrohr (32) gebildet ist, in welchem an dem Bildsensor (14) und/oder dem Leuchtmitteln angeschlossene Anschlussleitungen nach proximalseitig des Hohlschafts (2) geführt sind, wobei das Gehäuse (8) über eine Kulissensteuerung (46,50) mit einem in den Hohlschaft (2) in Längsrichtung des Hohlschaftes (2) bewegbaren Schub-Zug-Element bewegungsgekoppelt ist, welches von einem Rohr (36) gebildet wird, welches einen mit dem Innendurchmesser des Hohlschafts korrespondieren Außendurchmesser aufweist.

2. Schaftinstrument nach Anspruch 1, bei welchem eine außenliegende Außenseite (24) des Gehäuses (8) in der Stellung des Gehäuses (8) innerhalb des Innenlumens (22) des Hohlschafts (2) mit der Außenseite der Umfangswandung des Hohlschafts (2) bündig fluchtet.

3. Schaftinstrument nach Ansprüche 1 oder 2, bei welchem an der von der an der Umfangswandung des Hohlschafts (2) ausgebildeten Öffnung (20) abgewandten Außenseite (26) des Gehäuses (8) eine in Längsrichtung des Hohlschafts (2) verlaufende Ausnehmung (28) ausgebildet ist, in die der Träger eingreift.

4. Schaftinstrument nach einem der vorhergehenden Ansprüche, bei welchem das den Träger bildende Flachrohr (32) einen nierenförmigen Querschnitt aufweist.

5. Schaftinstrument nach einem der vorangehenden Ansprüche, bei welchem das Schub-Zug-Element zumindest einen quer zu seiner Bewegungsrichtung ausgerichteten Vorsprung (46) aufweist, der in eine an einer Außenseite des Gehäuses (8) ausgebildete und in radialer Richtung schräg verlaufende Führungsnut (50) eingreift.

6. Schaftinstrument nach einem der vorangehenen Ansprüche , bei welchem das das Schub-Zug-Element bildende Rohr (36) einen Längsschlitz (42) aufweist, wobei das Gehäuse (8) in den Längsschlitz (42) eingreift und an den beiden Längsseiten (44) des Längsschlitzes (42) ausgebildete Vorsprünge (46) in zwei voneinander abgewandten Außenseiten (48) des Gehäuses (8) ausgebildete Führungsnuten (5) eingreifen.

## Claims

1. A shank instrument, in particular medical-endoscopic shank instrument, with a hollow shank (2) and with at least one housing (8) which is integrated in the hollow shank (2) at the distal side and which has at least one electronic picture sensor (14) which is arranged therein for optically capturing a region which is located distally of the hollow shank (2) and/or has light means which are arranged therein for illuminating this region, wherein the housing (8) is movable in a straight-lined manner from a position, in which the picture sensor (14) and/or the light means are arranged within the inner lumen (22) of the hollow shank (2), into a position, in which the picture sensor (14) and/or the light means are arranged outside the inner lumen (22) of the hollow shank (2), wherein the housing (8) is displaceable through an opening (20) which is formed on the peripheral wall of the hollow shank (2) and at its inner-lying outer side (26) which is opposite and away from the opening (20) which is formed on the peripheral wall of the hollow shank (2) is supported on at least one elongate carrier which is guided in the hollow shank (2), is designed elastically resiliently transverse to its longitudinal extensions, extends essentially over the whole length of the hollow shank (2) and is fixed in the region of the proximal end of the hollow shank (2) or proximally of the hollow shank (2) **characterised in that** the carrier is formed by a flat tube (32), in which connection leads which are connected to the picture sensor (14) and/or to the light means are led proximally of the hollow shank (2), wherein the housing (8), via a cam guide (46, 50), is coupled in movement to a push-pull element which is movable into the hollow shank (2) in the longitudinal direction of the hollow shank (2), said push-pull element being formed by a tube (36) which has an outer diameter which corresponds to the inner diameter of the hollow shank.

2. A shank instrument according to claim 1, concerning which an outer-lying outer side (24) of the housing (8) is aligned in a flush manner with the outer side of the peripheral wall of the hollow shank (2) in the position of the housing (8) within the inner lumen (22) of the hollow shank (2).

3. A shank instrument according to claim 1 or 2, concerning which a recess (28) which runs in the longitudinal direction of the hollow shank (2) and into which the carrier engages is formed on the outer side (26) of the housing (8) which is away from the opening (20) which is formed on the peripheral wall of the hollow shank (2).

4. A shank instrument according to one of the preceding claims, concerning which the flat tube (32) which forms the carrier has a kidney-shaped cross section.

5. A shank instrument according to one of the preceding claims, concerning which the push-pull element comprises at least one projection (46) which is aligned transversely to its movement direction and which engages into a guide groove (50) which is formed on an outer side of the housing (8) and which runs obliquely in the radial direction.

6. A shank instrument according to one of the preceding claims, concerning which the tube (36) which forms the push-pull element comprises a longitudinal slot (42), wherein the housing (8) engages into the longitudinal slot (42) and projections (46) which are formed on the two longitudinal sides (44) of the longitudinal slot (42) engage into guide grooves (5) which are formed in two outer sides (48) of the housing (8) which are away from one another.

## Revendications

1. Instrument à tige, en particulier instrument médical d'endoscopie à tige, avec une tige creuse (2) et avec au moins un boîtier (8) intégré dans la tige creuse (2) du côté distal avec au moins un capteur d'image (14) électronique placé à l'intérieur de celui-ci pour la saisie optique d'une zone située du côté distal de la tige creuse (2) et/ou des moyens lumineux placés à l'intérieur de celui-ci pour l'éclairage de cette zone, le boîtier (8) étant mobile en ligne droite à partir d'une position, dans laquelle le capteur d'image (14) et /ou les moyens lumineux sont disposés à l'intérieur de la lumière interne (22) de la tige creuse (2), à une position dans laquelle le capteur d'images (14) et/ou les moyens lumineux sont disposés à l'extérieur de la lumière interne (22) de la tige creuse (2), le boîtier (8) étant déplaçable à travers une ouverture (20) formée sur la paroi périphérique de la tige creuse (2) et étant en appui, sur sa face extérieure (26) située à l'intérieur et opposée en sens opposé à l'ouverture (20) formée sur la paroi périphérique de la tige creuse (2), sur au moins un support allongé guidé dans la tige creuse (2) et formé élastiquement résilient transversalement à son étendue longitudinale qui s'étend sensiblement sur toute la longueur de la tige creuse (2) et est fixé dans la zone de l'extrémité proximale de la tige creuse (2) ou du côté proximal de la tige creuse (2),
**caractérisé en ce que** le support est constitué par un tuyau plat (32) dans lequel des lignes de raccordement raccordées au capteur d'image (14) et /ou aux moyens lumineux sont tirées vers le côté proximal de la tige creuse (2), le boîtier (8) étant accouplé en mouvement par une commande à coulisse (46, 50) à un élément de traction-poussée mobile dans la tige creuse (2), dans le sens longitudinal de la tige creuse (2), qui est constitué par un tube (36) qui présente un diamètre extérieur correspondant au diamètre intérieur de la tige creuse (2).

2. Instrument à tige selon la revendication 1, dans lequel une face extérieure (24) du boîtier (8) située à l'extérieur, dans la position du boîtier (8) à l'intérieur de la lumière interne (22) de la tige creuse (2), s'aligne exactement sur la face extérieure de la paroi périphérique de la tige creuse (2).

3. Instrument à tige selon la revendication 1 ou 2, dans lequel un évidement (28) s'étendant dans le sens de la longueur de la tige creuse (2) est formé sur la face extérieure (26) du boîtier (8), opposée à l'ouverture (20) formée dans la paroi périphérique de la tige creuse (2), dans lequel évidement le support s'engage.

4. Instrument à tige selon l'une des revendications précédentes, dans lequel le tuyau plat (32) formant le support présente une coupe transversale en forme de rein.

5. Instrument à tige selon l'une des revendications précédentes, dans lequel l'élément de traction-poussée présente au moins une avancée (46) dirigée transversalement à la direction de son mouvement, avancée qui s'engage dans une rainure de guidage (50) formée sur la face extérieure du boîtier (8) et s'étendant obliquement en direction radiale.

6. Instrument à tige selon l'une des revendications précédentes, dans lequel le tuyau (36) formant l'élément de traction-poussée présente une entaille longitudinale (42), le boîtier (8) s'engageant dans l'entaille longitudinale (42) et des avancées (46) formées sur les deux faces longitudinales (44) de l'entaille longitudinale (42) s'engageant dans deux rainures de guidage (5) formées dans deux faces extérieures (48) du boîtier (8) opposées l'une à l'autre.
